# FASCICULE DE BREVET EUROPEEN

(11) **EP 1 558 173 B1**
(45) Date de publication et mention de la délivrance du brevet: **20.02.2008**
(21) Numéro de dépôt: 03767898.4
(22) Date de dépôt: 04.11.2003
(51) Int. Cl.: A61F 2/00

(54) **PROTHESE DE RENFORT DE STRUCTURES TISSULAIRES**
Stützprothese für Gewebestrukturen
SUPPORT PROSTHESIS FOR TISSULAR STRUCTURES

(30) Priorité: 04.11.2002 FR 0213965
(43) Date de publication de la demande: 03.08.2005
(73) Titulaire: SOFRADIM PRODUCTION, 01600 Trévoux (FR)
(72) Inventeur: THERIN, Michel, F-69004 Lyon (FR); GRAVAGNA, Philippe, F-69450 Irigny (FR)
(74) Mandataire: Brédeville, Odile Marie
(86) Numéro de dépôt international: PCT/FR2003/003286
(87) Numéro de publication internationale: WO 2004/043294

(56) Documents cités:
- EP-A- 0 895 762
- FR-A- 2 724 563
- US-A- 4 603 695
- US-B1- 6 451 032

## Description

La présente invention concerne une prothèse de renfort de structures tissulaires, en particulier de tissus situés en dehors de la cavité péritonéale, à ce titre dits extrapéritonéaux.

Certains organes, ou portions d'organes, du corps humain, en particulier les organes extrapéritonéaux situés dans la cavité pelvienne, par exemple l'urètre, la vessie ou le rectum chez la femme, peuvent subir un déplacement anormal vers le bas appelé généralement prolapsus ou plus précisément cystocèle et rectocèle lorsqu'il intéresse respectivement la vessie ou le rectum. Ce déplacement intervient communément par suite du relâchement des moyens de fixation et de soutènement de l'organe. Ces phénomènes sont également appelés par l'homme de l'art : troubles de la statique pelvienne. Par ailleurs, suite à des hernies ou à des éventrations de la paroi abdominale, il est fréquent d'intervenir chirurgicalement pour traiter le déficit pariétal. Pour cela, le chirurgien a régulièrement recours à l'utilisation de prothèses de renfort et de soutien qu'on implante à l'endroit voulu. Ces prothèses de renfort sont généralement prévues pour une implantation définitive et sont de ce fait souvent constituées par exemple d'un support textile en un matériau non résorbable.

Les supports textiles sont intrinsèquement adhésiogènes et fibrosants quelle que soit la nature des tissus avec lesquels ils sont mis en contact. Cette propriété exprimée vis à vis des tissus de soutien (muscles, aponévroses, fascias etc..) constitue même un prérequis indispensable pour la qualité du résultat. En revanche, vis à vis d'autres structures plus fragiles, la présence d'un support textile lors de l'inflammation cicatricielle initiale favorise l'établissement de liens fibreux denses, là où n'existaient que des liens lâches tels que ceux que procurent les tissus conjonctifs interstitiels pour les organes extra-péritonéaux et où n'existait aucun lien pour les organes intra-péritonéaux. De ce fait, la nature poreuse des supports textiles est souvent à l'origine du développement d'adhérences et d'érosions post chirurgicales.

Pour remédier à ce problème, il a été proposé de rendre au moins une face de ces prothèses de renfort complètement lisse durant la phase inflammatoire initiale, et donc non propice à la génération de ces adhérences.

Ainsi, WO99/06080 décrit un support textile poreux destiné à une utilisation en chirurgie pariétale, dans la réparation des éventrations ou hernies, dont une face est recouverte superficiellement par une couche ou membrane plane résorbable, l'autre face étant laissée libre pour une intégration tissulaire intime et précoce.

WO96/08277 décrit une membrane transparente collagénique qui peut être déposée sur les deux faces d'un treillis textile synthétique.

De tels renforts peuvent représenter des solutions satisfaisantes dans le domaine de la chirurgie pariétale intrapéritonéale, qui concerne un site d'implantation se repéritonisant rapidement en surface, le péritoine néoformé constituant la meilleure protection des organes potentiellement au contact. Toutefois, dans le cas des traitements chirurgicaux pour le soutien de tissus ou la réparation des prolapsus, on peut avoir affaire à des tissus extrapéritonéaux qui sont particulièrement exposés car pouvant rentrer au contact des deux faces du renfort, par exemple vagin d'un côté, rectum ou vessie de l'autre, et ce d'autant plus qu'ils ne seront pas protégés à terme par la formation d'un néopéritoine. Ces tissus sont par exemple des viscères creux, comme la vessie, le vagin, l'utérus ou le rectum, ou des canaux naturels de l'organisme, comme l'urètre, l'oesophage, la trachée ou les vaisseaux sanguins, ou encore les nerfs, les tendons ou la dure mère. Pour de tels tissus dits fragiles, les liens fibreux postérieurs à l'inflammation cicatricielle initiale peuvent être aggravés d'érosion ou de fistules qui sont toujours indésirables car remettant en cause la physiologie normale de la structure impliquée. Dans ce cas, il est nécessaire, pendant la phase initiale de cicatrisation, à la fois de protéger ces tissus fragiles environnants tout en permettant le début de cicatrisation des tissus conjonctifs environnants de soutien (fascia, aponévroses, tissu conjonctif interstitiel), garant de la qualité à long terme du résultat de la reconstruction.

Par ailleurs, comme dans tout acte chirurgical, l'implantation d'un matériau non résorbable dans le corps humain présente certains risques infectieux. Ainsi, il peut arriver que le matériau implanté se trouve infecté par une contamination peropératoire du site d'implantation. Ce risque est d'autant plus marqué que les recommandations d'asepsie accompagnant tout acte chirurgical sont difficiles à mettre rigoureusement en oeuvre comme dans le cas des voies d'abord vaginales. A cet égard, il est connu que la surface des polymères synthétiques non résorbables est propice à l'adhésion des bactéries. L'adsorption ou la fixation d'une bactérie à un support non résorbable facilite la synthèse d'une coque de protection de la bactérie vis-à-vis des mécanismes naturels de défense ou antimicrobiens. Ainsi, pour un matériau donné, plus la surface développée de la prothèse est grande, plus l'adhésion bactérienne est importante. Or l'adhésion des bactéries à un support est une condition déclenchant et facilitant leur prolifération.

Ainsi, en vue d'optimiser l'acte chirurgical consistant à implanter des prothèses de renfort définitives, en particulier pour soutenir des tissus fragiles comme les tissus extrapéritonéaux, et notamment dans des -environnements où il est difficile de maintenir de strictes conditions d'asepsie, il serait particulièrement important de pouvoir réduire au maximum, pendant la phase initiale de cicatrisation, la surface développée de ces prothèses, accessible à la fois à ces tissus fragiles environnants et aux éventuelles bactéries. Ainsi, il serait souhaitable que ces prothèses présentent, au moins durant le temps de la phase initiale de cicatrisation, une surface physiquement non favorable à l'adhésion des bactéries et non dommageable aux tissus environnants la plus grande possible. De plus, un tel renfort n'offrirait aux bactéries éventuelles présentes qu'une surface temporaire biodégradable, facilitant en cela leur élimination par les cellules compétentes.

La Demanderesse a trouvé qu'un recouvrement spécifique, à savoir une enduction, de la surface solide développée d'un support textile par un film enveloppant en matériau résorbable hydrophile permettait de répondre aux attentes identifiées précédemment.

La présente invention a donc pour objet une prothèse composite de renfort d'une structure tissulaire, comprenant un support textile poreux comprenant un arrangement de fils composés chacun d'au moins un brin en matériau polymère non résorbable, ledit support textile définissant une texture microporeuse comprenant les interstices compris entre au moins deux fils aux endroits de contact d'un fil avec au moins un autre fil, caractérisée en ce que, dans au moins une zone protégée du support textile, un matériau résorbable hydrophile enduit le support textile, en formant un film enveloppant et pénétrant dans l'arrangement de fils, en obturant au moins la texture microporeuse, mais sans former une couche plane revêtant au moins une face du support textile.

La présente invention a également pour objet un procédé de préparation d'une prothèse composite de renfort d'une structure tissulaire, caractérisé en ce qu'il comprend les étapes suivantes :
- i) préparation d'une solution A d'un matériau résorbable hydrophile, à l'état fluide ou liquide,
- ii) imprégnation de solution A d'au moins une partie de la surface d'un support textile poreux, ledit support textile poreux comprenant un arrangement de fils composés chacun d'au moins un brin en matériau polymère non résorbable, ledit support textile définissant une texture microporeuse comprenant les interstices compris entre au moins deux fils aux endroits de contact d'un fil avec au moins un autre fil, ladite imprégnation ayant lieu jusqu'à obturation de la texture microporeuse, mais sans former une couche plane revêtant au moins une face dudit support textile,
- iii) séchage de la partie imprégnée du support textile.

Grâce à l'invention, la partie du support textile qui est enduite par le matériau résorbable est protégée durant la phase initiale de cicatrisation, à savoir, qu'elle n'est pas exposée aux cellules inflammatoires, comme les granulocytes, les monocytes, les macrophages ou encore les cellules géantes multi nucléées généralement activées par le geste chirurgical. Elle n'est pas exposée non plus aux bactéries éventuellement présentes. En effet, durant la phase initiale de cicatrisation, dont la durée peut varier de 5 à 10 jours environ, seul le matériau résorbable est accessible par les différents facteurs tels que les protéines, les enzymes, les cytokines ou les cellules de la lignée inflammatoire.

De plus, grâce à l'invention, durant le temps de digestion du matériau résorbable, les tissus fragiles environnants tels que les viscères creux par exemple, sont protégés, en particulier de la formation d'adhérences fibreuses sévères post-chirurgicales non souhaitées.

Un autre avantage de l'invention réside dans le fait qu'elle permet un début de cicatrisation des tissus conjonctifs environnants de soutien, comme les fascias, les aponévroses, les tissus conjonctifs interstitiels, au contact d'une surface hydrophile et résorbable, en elle-même favorable au développement rapide d'une couverture cellulaire faiblement inflammatoire. Ce début de cicatrisation de surface au contact du film de la prothèse recrée un plan régulier d'organisation de la fibrose et du tissu cicatriciel, qui facilite ultérieurement la restitution ad integrum des tissus clivés lors du geste chirurgical. Ainsi, après la résorption du film, l'envahissement de la porosité du support textile se fait directement par un tissu déjà partiellement libéré de sa composante inflammatoire.

Par support "textile poreux", on entend au sens de la présente invention, un support textile présentant des vides sous la forme d'interstices et/ou de volumes. Plus précisément, le support textile poreux est composé d'un arrangement de fils définissant une texture microporeuse et/ou une texture macroporeuse.

L'arrangement de fils dont il est question est un enchevêtrement ordonné, par exemple tissé selon toute armure appropriée, ou tricoté, ou encore désordonné, par exemple non tissé. Dans une forme privilégiée de réalisation de l'invention, l'arrangement de fils constitue une structure tricotée. Dans un tel cas, les interstices compris entre au moins deux fils à l'endroit de contact d'un fil avec au moins un autre fil appartiennent aux mailles de la structure tricotée. Les espaces vides définis entre les fils, en dehors de leurs endroits de contact, sont les espaces intermailles de la structure tricotée. Chaque fil dont il s'agit comprend au moins un brin ou filament, continu ou discontinu, en matériau polymère non résorbable; chaque fil peut comprendre d'autres fils ou filaments par exemple en matériau polymère résorbable.

Par "matériau polymère", on entend tout matériau, sous forme singulière ou d'alliage, comprenant un polymère synthétique ou naturel, obtenu par exemple par polymérisation ou copolymérisation. Le matériau polymère non résorbable selon l'invention peut être un polypropylène ou encore un polyester de type polyéthylène terephtalate.

La texture microporeuse ou microporosité comprend au moins les interstices compris entre au moins deux fils aux endroits de contact d'un fil avec au moins un autre fil. Dans le cas où au moins un fil comprend plusieurs brins en matériau polymère non résorbable, assemblés ou non, la texture microporeuse comprend en outre les interstices entre brins d'un même fil.

On peut donc définir la microporosité du support textile comme étant la somme i°) le cas échéant des interstices compris entre au moins deux brins au sein d'un même fil et ii°) des interstices compris entre au moins deux fils aux endroits de contact d'un fil avec au moins un autre fil.

La texture macroporeuse ou macroporosité comprend les volumes dont la surface S est définie par les espaces vides entre au moins deux fils, en dehors de leurs endroits de contact, et dont la hauteur H est définie par l'épaisseur du support textile. Selon la présente invention, on considère que le support textile est plat lorsqu'il constitue une structure bidimensionnelle, de préférence lorsque son épaisseur est inférieure ou égale à 5 fois le diamètre moyen des fils le constituant. Dans ce cas, on parlera de macroporosité bidimensionnelle ou de texture macroporeuse bidimensionnelle. Dans le cas où le support textile constitue une structure tridimensionnelle, de préférence lorsque l'épaisseur du support textile est strictement supérieure à 5 fois le diamètre moyen des fils constituant le support, on parlera de macroporosité tridimensionnelle ou de texture macroporeuse tridimensionnelle.

Dans une forme préférée de réalisation de l'invention, le support textile constitue une structure bidimensionnelle.

Le support textile est constitué d'un matériau polymère non résorbable communément utilisé en chirurgie. De préférence, ce matériau polymère non résorbable est choisi dans le groupe comprenant les polypropylènes, les polyesters comme les polyéthylène terephtalates, les polyamides et/ou leurs mélanges. Dans une forme privilégiée de réalisation de l'invention, ce matériau polymère est du polypropylène. Comme support textile à base de polypropylène convenant à la présente invention, on peut citer le produit vendu sous la dénomination commerciale « Parietene® » par la société Sofradim, le produit vendu sous la dénomination commerciale « Prolene® » par la société Ethicon ou encore le produit vendu sous la dénomination commerciale « Marlex® » par la société Bard.

Selon l'invention, on définit par « zone protégée » la zone du support textile enduite par le matériau résorbable hydrophile. Le matériau résorbable hydrophile est de préférence choisi dans le groupe constitué par les collagènes, les polysaccharides et leurs mélanges. Parmi les collagènes utilisables selon l'invention, on peut citer :
1) du collagène dont la structure hélicoïdale est au moins partiellement thermo-dénaturée, sans dégradation hydrolytique, dont la méthode de préparation est décrite dans WO99/06080,
2) du collagène natif, non chauffé, filmé, avec ou sans glycérine, réticulé par irradiation gamma ou par d'autres moyens chimiques ou physiques,
3) et/ou leurs mélanges.

Parmi les polysaccharides utilisables comme matériau hydrophile résorbable selon l'invention, on peut citer la cellulose oxydée, l'acide hyaluronique, l'amidon, le chitosan, les dextranes réticulés et/ou leurs mélanges. Tous ces matériaux sont bien connus de l'homme de l'art. Comme cellulose oxydée convenant à la présente invention, on peut citer le produit vendu sous la dénomination commerciale « Interceed® » par la société Ethicon. Comme acide hyaluronique convenant à la présente invention, on peut citer le produit vendu sous la dénomination commerciale « Hyalobarrier® » par la société Fidia Advanced Biopolymers, ou le produit vendu sous la dénomination commerciale « Seprafilm® » par la société Genzyme.

Le matériau résorbable hydrophile enduit la zone protégée du support textile en formant un film enveloppant et pénétrant dans l'arrangement de fils, qui obture la texture microporeuse, autrement dit au moins la microporosité du support, mais sans former une couche plane ou membrane épaisse revêtant au moins une face du support textile. Le film enrobe directement ou indirectement au moins chaque fil complètement, il réalise une enduction. De plus, dans le cas où chaque fil du support est constitué d'un seul brin, le film résorbable remplit et donc obture tous les interstices compris entre au moins deux fils aux endroits de contact d'un fil avec au moins un autre fil. Dans le cas où au moins un fil comporte plusieurs brins, assemblés ou non les uns aux autres, alors le film remplit et donc obture également tous les interstices entre brins d'un même fil.

Dans une forme préférée de réalisation de l'invention, le film de matériau résorbable est discontinu et préserve la texture macroporeuse du support textile. Le film enrobe alors chaque fil et laisse libres les volumes définissant la macroporosité. Ainsi, le renfort conserve une macroporosité prononcée pour un ancrage mécanique rapide et une réhabitation cellulaire immédiate. Une telle enduction par un film discontinu permet également de rigidifier temporairement le renfort et facilite sa manipulation par le chirurgien. Cette dernière propriété est particulièrement avantageuse en chirurgie laparoscopique.

De préférence, le film en matériau résorbable présente une épaisseur inférieure ou égale à 500 microns, et de préférence encore, allant de 10 à 100 microns.

Dans une autre forme de réalisation de l'invention, le film peut également obturer la macroporosité du support textile sur au moins une partie de la zone protégée. Selon l'invention, on définit par « zone obturée » la partie de la zone protégée pour laquelle la macroposité est obturée. Sur cette zone obturée, le film remplit et obture les volumes bidimensionnels ou tridimensionnels définissant la macroporosité. Il est donc continu. Dans une forme préférée de réalisation de l'invention, le film obture la macroporosité du support textile sur l'ensemble de la zone protégée.

La zone protégée peut indifféremment couvrir l'ensemble du support textile ou seulement une partie de ce support.

Dans une forme préférée de réalisation de l'invention, le support textile se présente sous la forme d'une pièce rectangulaire et la zone protégée s'étend sur une bande centrale de cette pièce. Une telle prothèse présente des zones à accroche immédiate pour une suspension efficace dès l'implantation, par rétraction des tissus conjonctifs interstitiels, des fascias et des aponévroses au niveau des bords libres des fils constituant le support textile, des zones relativement fibrosantes pour un ancrage mécanique durable au niveau de la partie non protégée du support textile, et des zones à intégration non agressive et peu fibrosante au niveau de la zone centrale protégée du renfort.

Grâce à l'invention, le praticien peut librement découper dans la prothèse des bandes de géométrie adaptée, et les disposer de façon à procurer un soutien non agressif, grâce à la zone protégée, vis-à-vis des structures tissulaires fragiles, et un ancrage rapide dans les zones non protégées à distance des structures fragiles. A titre d'exemple, les bandes découpées peuvent être à bords parallèles droits, de longueur et largeur modulables, à bords parallèles incurvés en arche, de longueur et largeur modulables, ou encore à bords non parallèles, renflées en région centrale, pour soutenir sur une grande surface les structures prolabées et plus effilées aux extrémités pour constituer des bandelettes de suspension d'ancrage.

La présente invention sera mieux comprise en se référant aux figures suivantes :
- la Figure 1 est une vue en coupe montrant la texture microporeuse d'un support textile composé de fils ayant chacun un seul brin ou filament ;
- la figure 2 est une vue en coupe à échelle agrandie montrant la texture microporeuse définie au sein d'un fil comportant plusieurs brins ;
- la Figure 3 est une vue de dessus montrant la macroporosité d'un support textile plat ;
- la Figure 4 est une vue en perspective montrant la macroporosité d'un support textile tridimensionnel ;
- la Figure 5 est une vue en coupe d'un support textile enduit par le film continu ;
- la Figure 6 est une vue en coupe d'un support textile enduit par un film discontinu ;
- la Figure 7 est une vue de dessus représentant-une prothèse de renfort de forme rectangulaire dont la zone protégée a la forme d'une bande centrale ;
- la Figure 8 est une vue de dessus de la prothèse de la figure 7 montrant différentes formes de bandelettes découpées dans la prothèse;
- la Figure 9 représente un dessin schématique de l'armure de tricotage d'un support textile convenant à la présente invention.

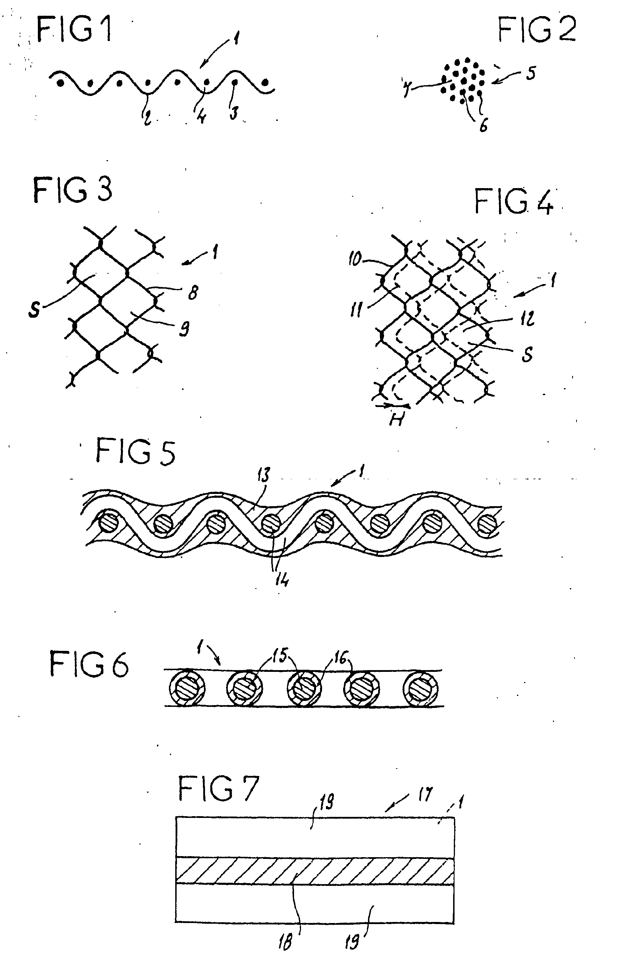

En se référant à la Figure 1, un support textile du type tissu poreux 1 est représenté, comprenant des fils de trame 2 et des fils de chaîne 3. Les interstices 4 définis dans ce cas entre les fils constituent la texture microporeuse.

La Figure 2 montre une coupe d'un fil 5 comportant plusieurs brins 6 continus ou discontinus, assemblés les uns aux autres, par exemple par torsion. Les interstices 7 définis entre les brins 6 au sein du fil 5 constituent aussi une texture microporeuse.

La Figure 3 montre des fils 8 tricotés constituant un support textile bidimensionnel 1. Les volumes 9 dont la surface S est définie par l'espace vide entre au moins deux fils en dehors de leurs endroits de contact, et dont la hauteur H est inférieure à environ 5 fois le diamètre moyen des fils 8 constituent une texture macroporeuse bidimensionnelle.

La Figure 4 montre un support textile tridimensionnel 1 composé de fils 10 constituant une première couche tricotée (ou tissée) plane et de fils 11 constituant une deuxième couche tricotée (ou tissée) plane parallèle à la première, séparée par une hauteur H; les première et seconde couches sont reliées entre elles par des boucles tricotées, par exemple. Les volumes 12 dont la surface S est définie par l'espace vide entre au moins deux fils en dehors de leurs endroits de contact et de hauteur H constituent une texture macroporeuse tridimensionnelle.

La Figure 5 montre un support textile 1 enduit par un film continu 13, et dont les fils 14 sont constitués de monofilaments.

La Figure 6 montre un support textile 1 composé de fils 15 à un seul brin et enduit par un film discontinu 16. Chaque brin est enrobé par le film 16. Les espaces vides entre les brins restent libres.

La Figure 7 montre une prothèse de renfort 17 de forme rectangulaire dont la zone protégée 18 représente une bande centrale, les parties latérales 19 du support textile 1 n'étant pas protégées.

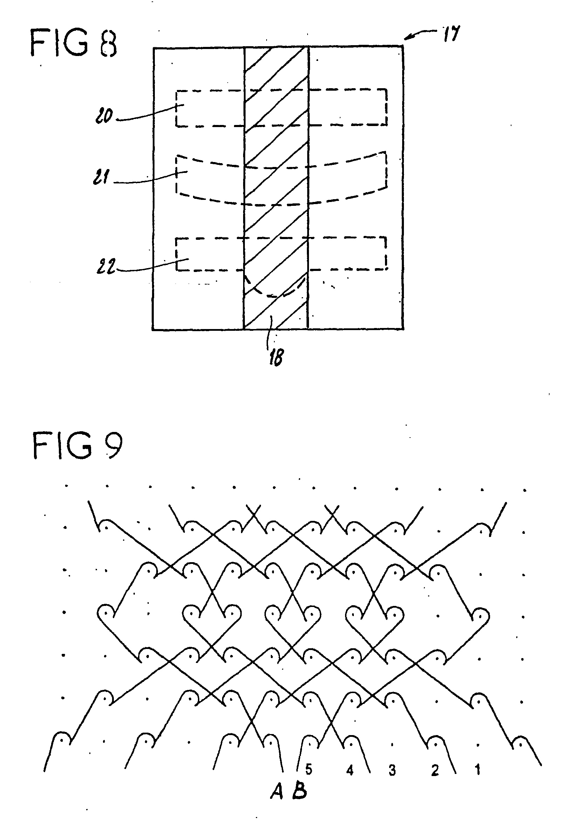

La Figure 8 montre une prothèse de renfort 17 de forme rectangulaire dont la zone protégée 18 a la forme d'une bande centrale, prothèse sur laquelle on a fait figurer les formes correspondant à:
- une bandelette 20 à bords parallèles, dont la partie centrale est une zone protégée,
- une bandelette 21 à bords parallèles incurvés en arche, dont la partie centrale est une zone protégée,
- une bandelette 22 à bords non parallèles, dont la partie centrale renflée est une zone protégée et dont les parties latérales effilées sont non protégées.

Ces bandelettes peuvent être découpées par le chirurgien directement sur le site opératoire et constituent en elles-mêmes des prothèses selon l'invention.

Une prothèse composite de renfort selon l'invention peut être préparée selon le procédé comprenant les étapes suivantes :
- i) préparation d'une solution A d'un matériau résorbable hydrophile, à l'état fluide ou liquide,
- ii) imprégnation de solution A d'au moins une partie de la surface d'un support textile poreux, ledit support textile poreux comprenant un arrangement de fils composés chacun d'au moins un brin en matériau polymère non résorbable, ledit support textile définissant une texture microporeuse comprenant les interstices compris entre au moins deux fils aux endroits de contact d'un fil avec au moins un autre fil, ladite imprégnation ayant lieu jusqu'à obturation de la texture microporeuse, mais sans former une couche plane revêtant au moins une face plane dudit support textile,
- iii) séchage de la partie imprégnée du support textile.

Le procédé de préparation de la prothèse selon l'invention est simple et rapide. Ainsi, il ne comprend qu'une seule étape d'imprégnation de la partie à protéger du support textile. L'étape d'imprégnation peut se faire par immersion de ladite partie de la surface du support textile dans la solution A ou par pulvérisation, de préférence à l'aide d'un pochoir ou d'un masque de protection, de la solution A sur ladite partie de la surface du support textile. La solution d'imprégnation A doit être suffisamment fluide pour qu'elle imprègne le support textile jusqu'à obturation de la microporosité. Cette solution peut être chauffée à une température inférieure à 50°C. De préférence, afin de faciliter cette étape d'imprégnation, la solution A présente une viscosité inférieure ou égale à 1000 centipoises, et de préférence encore allant de 50 à 200 centipoises, cette viscosité étant mesurée à l'aide d'un viscosimètre CONTRAVES -TV par exemple à la température choisie inférieure 50°C. Les parties du support textile qui ne sont pas destinées à être enduites peuvent être recouvertes d'une membrane protectrice durant l'étape ii) d'imprégnation. Dans le cas où la zone enduite représente une bande centrale du support textile, on peut aussi préalablement plier le support en forme de U, la partie à enduire se trouvant être la barre horizontale du U, et tremper, ainsi la partie à enduire dans la solution A tout en préservant les parties latérales (barres verticales du U).

Dans le cas où l'on souhaite enduire la totalité du support textile, on trempe simplement le support textile en entier dans la solution A et on le laisse sécher. La zone protégée a alors une surface recouvrant entièrement celle du support textile.

L'étape d'imprégnation peut être répétée si on souhaite augmenter la quantité de matière enduite.

Le séchage de la partie imprégnée du support textile peut se faire en laissant reposer le support sous un flux d'air propre à température ambiante ou encore dans une enceinte thermostatée à une température maximale adaptée au produit résorbable, par exemple inférieure ou égale à 30°C pour le collagène, inférieure ou égale à 60°C pour les polysaccharides.

Ainsi, la prothèse de renfort selon l'invention peut être fabriquée en découpant d'abord un support textile à la forme et aux dimensions voulues, que l'on enduit ensuite totalement ou partiellement selon le procédé décrit ci-dessus afin d'obtenir la prothèse définitive que l'on souhaite implanter dans le corps du patient.

Une autre façon de fabriquer une prothèse selon l'invention est d'enduire préalablement une partie, par exemple une bande centrale, d'un support textile de forme donnée, par exemple rectangulaire, selon le procédé décrit ci-dessus, par exemple en pliant le support en forme de U, et de découper ensuite dans ce support partiellement enduit une prothèse selon une géométrie déterminée de sorte que dans sa configuration finale, cette prothèse présente par exemple une zone centrale protégée et des zones latérales non protégées à accroche immédiate.

La prothèse de renfort selon l'invention peut s'appliquer à toute structure tissulaire du corps. De préférence, cette structure tissulaire est un tissu extrapéritonéal.

### EXEMPLE 1:

On réalise un support textile dont l'arrangement de fils constitue un tricot réalisé par tricotage en chaîne de deux nappes de fils, conformément au dessin schématique de la Figure 9. Dans cette figure, chaque nappe de fils est identifiée par une lettre, A ou B, le schéma en lui-même utilisant un système de description du tricot à réaliser tout à fait habituel et compréhensible pour l'homme du métier, et qui ne sera pas décrit plus en détails ici. Le support textile est tricoté sur un métier de type chaîne avec deux barres à passettes.

Les deux barres correspondant aux fils A et B sont enfilées une passette pleine, une passette vide et se déplacent symétriquement afin d'obtenir des jours en quinconce. Le support textile final peut ensuite être stabilisé simplement par passage au four à une température comprise entre environ 110°C et environ 150°C.

Les fils sont des monofilaments de polypropylène dont le diamètre peut varier de 0,08 à 0,18 mm.

Le support textile ainsi préparé en utilisant un fil de 0.10 mm de diamètre présente les caractéristiques physico-mécaniques suivantes : son poids est de 38 g/m², son épaisseur est de 0.4 mm, la surface moyenne des pores, mesurée en microscopie optique ou électronique, est de 1.5x1.7 mm², la porosité moyenne, mesurée par le calcul du rapport entre la densité du matériau constitutif et le volume total du textile est de 89%, la résistance à la rupture, mesurée selon la norme ISO13934-1, est de 146 N en chaîne et 146 N en trame, la résistance à la déchirure, mesurée selon la norme ISO4674-A2 est de 26 N en chaîne et 23.6 N en trame, l'éclatométrie, mesurée selon la norme ISO13938-1, est de 617 Kpa, l'élasticité sous 10 daN, exprimée en pourcentage de déformation sous la charge indiquée (10daN) dans le test selon IS013934-1 est de 53% en chaîne et 62% en trame.

Il est possible d'intégrer à ce textile des zones, dans le sens de la fabrication textile, obtenues par tricotage de fils et/ou d'armure différentes de façon à obtenir des zones à caractéristiques physiques (tailles des pores, densité, épaisseur) et mécaniques (résistance au tuilage, élasticité) adaptées à la présente invention.

On prépare une solution comprenant du collagène modifié par coupure oxydative et chauffage, du polyéthylène glycol et de la glycérine. Les concentrations pondérales en collagène, en polyéthylène glycol et en glycérine sont de préférence respectivement comprises entre environ 2 et 9% pour le collagène, environ 0,6 et 3% pour le polyéthylène glycol, environ 0,3 et 1,2% pour la glycérine.

- Cette solution présente une viscosité allant de 50 à 200 centipoises. On immerge une bande centrale du support textile dans cette solution, puis on le retire et on le laisse gélifier 30 minutes. On répète cette opération autant de fois qu'il est nécessaire pour obtenir un film de densité 0.133 g/cm².

Après maturation, on stérilise le tout par de l'oxyde d'éthylène.

On obtient un support textile dont la bande centrale est obturée.

### EXEMPLE 2 :

A partir du même support textile et de la même solution que décrits dans l'exemple 1, on pulvérise la solution sur la bande centrale du support textile qu'on souhaite protéger. La pulvérisation est pratiquée sur une face du textile. Après 30minutes de maturation du gel formé à la surface du textile, on pulvérise la solution sur l'autre face. Après la deuxième pulvérisation, on laisse sécher l'ensemble sous flux d'air stérile. On pulvérise la quantité de solution nécessaire pour obtenir un film de densité finale 0.133 g/cm². Après maturation, on stérilise le tout par de l'oxyde d'éthylène.

On obtient une pièce composite intermédiaire dont la bande centrale est une zone obturée selon l'invention et dont les parties latérales sont exemptes de matériau résorbable.

## Revendications

1. Prothèse composite de renfort (17) d'une structure tissulaire, comprenant un support textile poreux (1) comprenant un arrangement de fils (8, 10, 11, 14, 15) composés chacun d'au moins un brin (6) en matériau polymère non résorbable, ledit support textile définissant une texture microporeuse comprenant les interstices (4) compris entre au moins deux fils aux endroits de contact d'un fil avec au moins un autre fil, **caractérisée en ce que**, dans au moins une zone protégée (18) du support textile, un matériau résorbable hydrophile enduit le support textile, en formant un film enveloppant et pénétrant dans l'arrangement de fils, en obturant au moins la texture microporeuse, mais sans former une couche plane revêtant au moins une face du support textile.

2. Prothèse selon la revendication 1, **caractérisée en ce que** le support textile (1) définit également une texture macroporeuse, comprenant les volumes (9, 12) dont la surface S est définie par les espaces vides entre au moins deux fils en dehors de leurs endroits de contact et dont la hauteur H est définie par l'épaisseur du support textile, et **en ce que** le film de matériau résorbable est discontinu (16) et préserve la texture macroporeuse du support textile.

3. Prothèse selon la revendication 1 ou 2, **caractérisée en ce que** le support textile (1) constitue une structure bidimensionnelle.

4. Prothèse selon l'une quelconque des revendications précédentes, **caractérisée en ce que** le film (13, 16) présente une épaisseur inférieure ou égale à 500 microns.

5. Prothèse selon la revendication 4, **caractérisée en ce que** le film présente une épaisseur allant de 10 à 100 microns.

6. Prothèse selon l'une quelconque des revendications précédentes, **caractérisée en ce que** au moins un fil (5) comprend plusieurs brins (6) en matériau polymère non résorbable, et la texture microporeuse comprend en outre les interstices (7) entre brins d'un même fil.

7. Prothèse selon la revendication 1 ou 2, **caractérisée en ce que** la zone protégée (18) a une surface recouvrant entièrement celle du support textile (1).

8. Prothèse selon la revendication 1 ou 2, **caractérisée en ce que** le support textile (1) présente la forme d'une pièce rectangulaire et la zone protégée (18) s'étend selon une bande centrale de ladite pièce.

9. Prothèse selon l'une quelconque des revendications précédentes, **caractérisée en ce qu'**elle a la forme d'une bandelette (20) à bords parallèles, dont la partie centrale est une zone protégée.

10. Prothèse selon l'une quelconque des revendications 1 à 8, **caractérisée en ce qu'**elle a la forme d'une bandelette (21) à bords parallèles incurvés en arche, dont la partie centrale est une zone protégée.

11. Prothèse selon l'une quelconque des revendications précédentes, **caractérisée en ce qu'**elle a la forme d'une bandelette (22) à bords non parallèles, dont la partie centrale renflée est une zone protégée et dont les parties latérales effilées sont non protégées.

12. Prothèse selon l'une quelconque des revendications 2 à 11, **caractérisée en ce que** le film obture la macroporosité du support textile sur au moins une partie de la zone protégée.

13. Prothèse selon la revendication précédente, **caractérisée en ce que** le film obture la macroporosité du support textile sur l'ensemble de la zone protégée.

14. Prothèse selon l'une quelconque des revendications précédentes, **caractérisée en ce que** le matériau résorbable est choisi dans le groupe constitué par les collagènes, les polysaccharides et leurs mélanges.

15. Prothèse selon l'une quelconque des revendications précédentes, **caractérisée en ce que** l'arrangement de fils constitue une structure tricotée.

16. Prothèse selon la revendication 15, **caractérisée en ce que** les interstices compris entre au moins deux fils à l'endroit de contact d'un fil avec au moins un autre fil appartiennent aux mailles de la structure tricotée.

17. Prothèse selon la revendication 15 ou 16, **caractérisée en ce que** les espaces vides définis entre les fils, en dehors de leurs endroits de contact, sont les espaces intermailles de la structure tricotée.

18. Procédé de préparation d'une prothèse composite de renfort (17) d'une structure tissulaire, **caractérisé en ce qu'**il comprend les étapes suivantes :
- i) préparation d'une solution A d'un matériau résorbable hydrophile, à l'état fluide ou liquide,
- ii) imprégnation de solution A d'au moins une partie de la surface d'un support textile poreux, ledit support textile poreux comprenant un arrangement de fils composés chacun d'au moins un brin en matériau polymère non résorbable, ledit support textile définissant une texture microporeuse comprenant les interstices compris entre au moins deux fils aux endroits de contact d'un fil avec au moins un autre fil, ladite imprégnation ayant lieu jusqu'à obturation de la texture microporeuse, mais sans former une couche plane revêtant au moins une face dudit support textile,
- iii) séchage de la partie imprégnée du support textile.

19. Procédé selon la revendication 18, **caractérisé en ce que** l'étape d'imprégnation se fait par immersion de ladite partie de la surface du support textile dans la solution A.

20. Procédé selon la revendication 18, **caractérisé en ce que** l'étape d'imprégnation se fait par pulvérisation de la solution A sur ladite partie de la surface du support textile.

21. Procédé selon l'une quelconque des revendications 18 à 20, **caractérisé en ce que** la solution A présente une viscosité inférieure ou égale à 1000 centipoises.

## Claims

1. A composite prosthesis (17) for reinforcement of a tissue structure, comprising a porous textile support (1) which includes an arrangement of threads (8, 10, 11, 14, 15) each composed of at least one filament (6) of nonabsorbable polymer material, said textile support defining a microporous texture comprising the interstices (4) located between at least two threads at the sites of contact of one thread with at least one other thread, wherein, in at least one protected zone (18) of the textile support, a hydrophilic absorbable material coats the textile support, forming a film enveloping and penetrating into the arrangement of threads, occluding at least the microporous texture, but without forming a plane layer covering at least one face of the textile support.

2. The prosthesis as claimed in claim 1, wherein the textile support (1) also defines a macroporous texture comprising the volumes (9, 12) whose surface S is defined by the empty spaces between at least two threads away from their sites of contact, and whose height H is defined by the thickness of the textile support, and wherein the film of absorbable material is noncontinuous (16) and preserves the macroporous texture of the textile support.

3. The prosthesis as claimed in claim 1 or 2, wherein the textile support (1) constitutes a two-dimensional structure.

4. The prosthesis as claimed in any one of the preceding claims, wherein the film (13, 16) has a thickness of less than or equal to 500 microns.

5. The prosthesis as claimed in claim 4, wherein the film has a thickness of from 10 to 100 microns.

6. The prosthesis as claimed in any one of the preceding claims, wherein at least one thread (5) comprises several filaments (6) of nonabsorbable polymer material, and the microporous texture additionally comprises the interstices (7) between filaments of the same thread.

7. The prosthesis as claimed in claim 1 or 2, wherein the protected zone (18) has a surface completely covering that of the textile support (1).

8. The prosthesis as claimed in claim 1 or 2, wherein the textile support (1) has the shape of a rectangular part and the protected zone (18) extends along a central band of said part.

9. The prosthesis as claimed in any one of the preceding claims, wherein it is in the shape of a strip (20) with parallel edges, the central part being a protected zone.

10. The prosthesis as claimed in any one of claims 1 through 8, wherein it is in the shape of a strip (21) with parallel edges which are curved in an arch, the central part being a protected zone.

11. The prosthesis as claimed in any one of the preceding claims, wherein it is in the shape of a strip (22) with nonparallel edges, the bulged central part being a protected zone and the narrower lateral parts being nonprotected.

12. The prosthesis as claimed in any one of claims 2 through 11, wherein the film occludes the macroporosity of the textile support over at least part of the protected zone.

13. The prosthesis as claimed in the preceding claim, wherein Lhe film occludes the macroporosity of the textile support over the whole of the protected zone.

14. The prosthesis as claimed in any one of the preceding claims, wherein the absorbable material is chosen from the group formed by collagens, polysaccharides, and their mixtures.

15. The prosthesis as claimed in any one of the preceding claims, wherein the arrangement of threads constitutes a knitted structure.

16. The prosthesis as claimed in claim 15, wherein the interstices located between at least two threads at the site of contact of one thread with at least one other thread belong to the meshes of the knitted structure.

17. The prosthesis as claimed in claim 15 or 16, wherein the empty spaces defined between the threads, away from their sites of contact, are the intermesh spaces of the knitted structure.

18. A process for preparing a composite prosthesis (17) for reinforcement of a tissue structure, said process comprising the following steps:
- i) preparing a solution A of a hydrophilic absorbable material, in the fluid or liquid state,
- ii) impregnating at least part of the surface of a porous textile support with solution A, said porous textile support comprising an arrangement of threads each composed of at least one filament of nonabsorbable polymer material, said textile support defining a microporous texture which includes the interstices located between at least two threads at the sites of contact of one thread with at least one other thread, said impregnating taking place until occlusion of the microporous texture, but without forming a plane layer covering at least one face of the textile support,
- iii) drying the impregnated part of the textile support.

19. The process as claimed in claim 18, wherein the impregnation step is done by immersing said part of the surface of the textile support in solution A.

20. The process as claimed in claim 18, wherein the impregnation step is done by spraying solution A onto said part of the surface of the textile support.

21. The process as claimed in any one of claims 18 through 20, wherein solution A has a viscosity of less than or equal to 1000 centipoises.

## Patentansprüche

1. Kompositverstärkungsprothese (17) zum Verstärken einer Gewebsstrukrur, mit einem porösen textilen Träger (1), der eine Anordnung von Fäden (8, 10, 11, 14, 15) aufweist, die jeweils aus zumindest einer Eleimentarfaser (6) aus einem nichtresorbierbaren polymeren Material aufgebaut sind, wobei der textile Träger ein mikroporöses Gefüge definiert, das Zwischenräume (4) zwischen zumindest zwei Fäden an Berührstellen eines Fadens mit zumindest einem weiteren Faden aufweist, **dadurch gekennzeichnet, dass** in zumindest einer geschützten Zone (18) des textilen Trägers ein hydrophiles resorbierbares Material den textilen Träger überzicht, indem es einen Film bildet, der in die Anordnung von Fäden eindringt und diese umhüllt und **dadurch** zumindest das mikroporöse Gefüge verschließt, jedoch ohne eine ebene Schicht zu bilden, die zumindest eine Seite des textilen Trägers bedeckt.

2. Prothese nach Anspruch 1, **dadurch gekennzeichnet, dass** der textile Träger (1) außerdem ein makroporöses Gefüge definiert, das Volumina (9, 12) aufweist, deren Oberfläche S durch die leeren Räume zwischen zumindest zwei Fäden außerhalb ihrer Berührstellen definiert ist und deren Höhe H durch die Dicke des textilen Trägers definiert ist, und dass der Film aus resorbierbarem Material unterbrochen (16) ist und das makroporöse Gefüge des textilen Trägers schützt.

3. Prothese nach Anspruch 1 oder 2, **dadurch gekennzeichnet, dass** der textile Träger (1) eine zweidimensionale Struktur bildet.

4. Prothese nach einem der vorhergehenden Ansprüche, **dadurch gekennzeichnet, dass** der Film (13, 16) eine Dicke von weniger als oder gleich 500 Mikron aufweist.

5. Prothese nach Anspruch 4, **dadurch gekennzeichnet, dass** der Film eine Dicke von 10 bis 100 Mikron aufweist.

6. Prothese nach einem der vorhergehenden Ansprüche, **dadurch gekennzeichnet, dass** zumindest ein Faden (5) mehrere Elementarfasern (6) aus nicht-resorbierbarem polymeren Material aufweist, und das mikroporöse Gefüge außerdem die Zwischenräume (7) zwischen Elementarfasern eines selben Fadens aufweist.

7. Prothese nach Anspruch 1 oder 2, **dadurch gekennzeichnet, dass** die geschützte Zone (18) eine Oberfläche aufweist, die diejenige des textilen Trägers (1) vollständig bedeckt.

8. Prothese nach Anspruch 1 oder 2, **dadurch gekennzeichnet, dass** der textile Träger (1) die Form eines rechteckigen Stücks aufweist und sich die geschützte Zone (18) entlang eines mittigen Streifens des Stückes erstreckt.

9. Prothese nach einem der vorhergehenden Ansprüche, **dadurch gekennzeichnet, dass** sie die Form eines kleinen Streifens (20) mit parallelen Rändern aufweist, dessen mittiger Teil eine geschützte Zone ist.

10. Prothese nach einem der Ansprüche 1 bis 8, **dadurch gekennzeichnet, dass** sie die Form eines kleinen Streifens (21) mit bogenförmig gekrümmten parallelen Rändern aufweist, dessen mittiger Teil eine geschützte Zone ist.

11. Prothese nach einem der vorhergehenden Ansprüche, **dadurch gekennzeichnet, dass** sie die Form eines kleinen Streifens (22) mit nicht parallelen Rändern aufweist, dessen bauchiger mittiger Teil eine geschützte Zone ist und dessen schmale seitlichen Teile nicht geschützt sind.

12. Prothese nach einem der Ansprüche 2 bis 11, **dadurch gekennzeichnet, dass** der Film die Makroporosität des textilen Trägers Über zumindest einen Teil der geschützten Zone verschließt.

13. Prothese nach dem vorhergehenden Anspruch, **dadurch gekennzeichnet, dass** der Film die Makroporosität des textilen Trägers über die Gesamtheit der geschützten Zone verschließt.

14. Prothese nach einem der vorhergehenden Ansprüche, **dadurch gekennzeichnet, dass** das resorbierbare Material aus der Gruppe ausgewählt ist, die durch die Kollagene, Polysacharide und ihre Gemische gebildet ist.

15. Prothese nach einem der vorhergehenden Ansprüche, **dadurch gekennzeichnet, dass** die Anordnung von Fäden eine gewirkte Struktur bildet.

16. Prothese nach Anspruch 15, **dadurch gekennzeichnet, dass** die Zwischenraume im Bereich zwischen zumindest zwei Fäden an der Berührstelle eines Fadens mit zumindest einem weiteren Faden zu den Maschen der gewirkten Struktur gehören.

17. Prothese nach Anspruch 15 oder 16, **dadurch gekennzeichnet, dass** die leeren Räume, die zwischen den Fäden außerhalb ihrer Berührstellen definiert sind, Maschenzwischenräume der gewirkten Struktur sind.

18. Verfahren zum Präparieren einer Kompositverstärkungsprothese (17) zum Verstärken einer Gewebsstruktur, **dadurch gekennzeichnet, dass** es die folgenden Schritte aufweist:
- i) Präparieren einer Lösung A aus einem hydrophilen resorbierbaren Material im flüssigen oder fluiden Zustand,
- ii) Imprägnieren zumindest eines Teils der Oberfläche eines porösen textilen Trägers mit der Lösung A, wobei der poröse textile Träger eine Anordnung von Fäden aufweist, die jeweils aus zumindest einer Elementarfaser aus nicht-resorbierbarem polymeren Material aufgebaut sind, wobei der textile Träger ein mikroporöses Gefüge definiert, das Zwischenräume im Bereich zwischen zumindest zwei Fäden an Berührstellen eines Fadens mit zumindest einem weiteren Faden aufweist, wobei das imprägnieren bis zum Verschließen des mikroporösen Gefüges erfolgt, ohne jedoch eine ebene Schicht zu bilden, die zumindest eine Seite des textilen Trägers bedeckt,
- iii) Trocknen des imprägnierten Teils des textilen Trägers.

19. Verfahren nach Anspruch 18, **dadurch gekennzeichnet, dass** der Schritt des Imprägnierens durch Eintauchen des Teils der Oberfläche des textilen Trägers in die Lösung A durchgeführt wird.

20. Verfahren nach Anspruch 18, **dadurch gekennzeichnet, dass** der Schritt des Imprägnierens durch Zerstäuben der Lösung A auf den Teil der Oberfläche des textilen Trägers durchgeführt wird.

21. Verfahren nach einem der Ansprüche 18 bis 20, **dadurch gekennzeichnet, dass** die Lösung A eine Viskosität von weniger als oder gleich 1000 Zentipoise aufweist.
